# EUROPÄISCHE PATENTANMELDUNG

(11) **EP 1 096 158 A2**
(43) Veröffentlichungstag der Anmeldung: **02.05.2001**
(21) Anmeldenummer: 00122515.0
(22) Anmeldetag: 14.10.2000
(51) Int. Cl.: F16B 7/18

(54) **Klemmbefestigung**

(30) Priorität: 27.10.1999 DE 29918829 U
(71) Anmelder: Lehmann, Angelika Franziska, 84048 Mainburg (DE)
(72) Erfinder: Lehmann, Angelika Franziska, 84048 Mainburg (DE)
(74) Vertreter: Graf, Helmut, Dipl.-Ing.

(57) **Zusammenfassung**

Die Erfindung bezieht sich auf eine neuartige Ausbildung einer Klemmbefestigung zur Befestigung eines Funktionselementes, beispielsweise einer Auflage, einer Ablage, eines Tisches oder eines Handgriffs.

## Beschreibung

Die Erfindung bezieht sich auf eine Klemmbefestigung gemäß Oberbegriff Patentanspruch 1 sowie auf Funktionselemente mit einer solchen Klemmbefestigung gemäß Oberbegriff Patentansprüche 8 bis 11, insbesondere zur Verwendung im Heil- und Pflegebereich.

Speziell im Heil- und Pflegebereich ist es vielfach wünschenswert, an vorhandenen Einrichtungen Funktionselementen, beipsielsweise Auf- und Ablagen für Extremitäten des menschlichen Körpers (Arme oder Beine), tischförmige Ablagen zum Abstellen von Gegenständen, aber auch andere Funktionselemente, beispielsweise Handgriffe usw. zu befestigen, wobei die Art und Anzahl der Funktionselementen dem jeweiligen Bedarf entsprechend sehr unterschiedlich sein kann. So ist es beispielsweise vielfach sinnvoll an Infusionsständern derartig Funktionselemente zumindest vorübergehend vorzusehen.

Aufgabe der Erfindung ist es, eine Klemmbefestigung aufzuzeigen, mit der in besonders einfacher Weise das Anbringen von Funktionselementen an stangenartigen Elementen, insbesondere an stangenartigen Elementen von Einrichtungen des Heil- und Pflegebereichs möglich ist.

Zur Lösung dieser Aufgabe ist eine Klemmbefestigung entsprechend dem Patentanspruch 1 ausgebildet. Funktionselemente mit einer solchen Klemmbefestigung sind entsprechend den Patentansprüchen 8 bis 11 ausgeführt.

Weiterbildungen der Erfindung sind Gegenstand der Unteransprüche. Die Erfindung wird im Folgenden anhand der Figuren an Ausführungsbeispielen näher erläutert. Es zeigen:
- Fig. 1 und 2: in vereinfachter Darstellung und in Seitenansicht bzw. in Draufsicht eine mit einer erfindungsgemäßen Klemmbefestigung ausgestattete Ablage;
- Fig. 3 und 4: in Teildarstellung einen mit der erfindungsgemäßen Klemmbefestigung ausgestatteten Tisch bzw. Handgriff;
- Fig. 5: in vereinfachter Darstellung und in Draufsicht als weitere mögliche Ausführung einen mit mehreren Klemmvorrichtungen ausgestatteten Tisch;
- Fig. 6: in Seitendarstellung und teilweise im Schnitt eine weitere mögliche Ausführungsform der erfindungsgemäßen Klemmbefestigung;
- Fig. 7: eine Draufsicht auf den Fixierkloben bzw. die Klemm-Mutter des Klemmstückes der Figur 6.

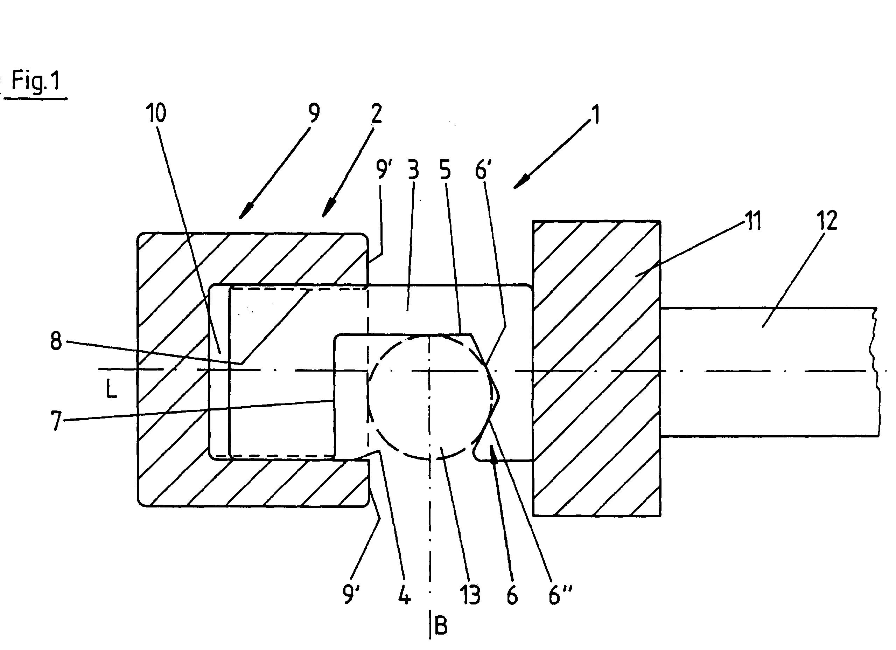

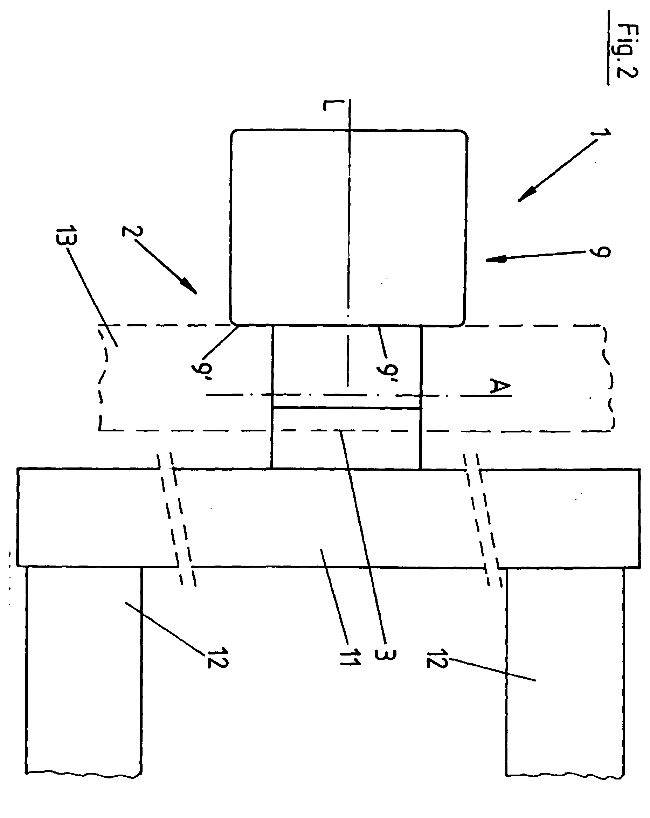

Die in den Figuren 1 und 2 allgemein mit 1 bezeichnete Ab- bzw. Auflage für Extremitäten eines menschlichen Körpers, beispielsweise für einen Fuß oder Arm ist zur Befestigung an einem nicht dargestellten Infusionsständer bestimmt.

Die Ablage 1 umfaßt u.a. eine Klemmbefestigung 2, die im wesentlichen aus einem beispielsweise aus Metall gefertigten Klemmblock oder -stück 3 besteht, welches bei der dargestellten Ausführungsform einen kreiszylinderförmigen Außendurchmesser aufweist und mit einer seitlichen Ausnehmung 4 versehen ist.

Die Ausnehmung 4 erstreckt sich entlang einer die Längsachse L des Klemmstücks 3 konzentrisch umschließenden Umfangslinie über einen Winkelbereich von etwas mehr als 180° und ist so ausgeführt, daß sie in einer Achsrichtung A, die sich senkrecht zur Längsachse L erstreckt und auch in einer Ebene senkrecht zu dieser Längsachse sowie senkrecht zur Zeichenebene der Figur 1 liegt, beidseitig eine größere Öffnung und dazwischenliegend eine Öffnung aufweist, die in Achsrichtung L des Klemmstückes 3 eine reduzierte Breite besitzt.

Die Ausnehmung 4 besitzt hierfür in Ebenen senkrecht zur Achse A einen Querschnitt derart, daß sich dieser Querschnitt ausgehend von der geschlossenen Seite bzw. Bodenfläche 5 der Ausnehmung in Richtung der Längsachse L zunächst verbreitert und dann wieder verengt. Dementsprechend ist die an die Bodenfläche 5 anschließende eine Seitenfläche 6 der Ausnehmung 4 konkav ausgebildet, und zwar mit zwei Abschnitten 6' und 6'', von denen der Abschnitt 6' an die Bodenfläche 5 anschließt und so verläuft, daß im Bereich dieses Abschnittes die Breite des Querschnittes in Richtung der Längsachse 1 zunimmt und im Bereich des anschließenden Abschnittes 6'' wieder abnimmt.

Die der Seitenfläche 6 gegenüberliegende und ebenfalls an die Bodenfläche 5 anschließende Seitenfläche 7 ist bei der dargestellten Ausführungsform im wesentlichen plan ausgebildet und liegt in einer Ebene senkrecht zur Längsachse L. Ebenfalls plan ausgebildet sind die Bodenfläche 5 sowie die Abschnitte 6' und 6'' der Seitenfläche 6. Die Bodenfläche 5 liegt in einer Ebene parallel zur Längsachse L und parallel zur Achse A. Die Abschnitte 6' und 6'' liegen jeweils in Ebenen parallel zur Achse A, die allerdings mit der Längsachse L jeweils einen Winkel kleiner als 90° einschließen.

Auf dem der Seitenfläche 7 benachbarten Abschnitt ist das Klemmstück 3 mit einem Außengewinde 8 versehen, auf dem eine napf- oder hutartige Klemm-Mutter 9 mit einem Innengewinde einer Gewindebohrung 10 geführt ist. Das dortige Ende des Klemmstückes 3 ist von der Gewindebohrung 10 aufgenommen. Am anderen, gegenüberliegenden Ende des Klemmstückes 3 ist ein balkenartiges Tragstück 11 befestigt, welches radial zur Längsachse L liegt und beidendig über das Klemmstück 3 radial wegsteht. An jedem Ende des Tragstückes 11 ist ein vorzugsweise gepolstertes walzenartiges Auflage- oder Abstützelement 12 befestigt, und zwar derart, daß jedes Element 12 mit seiner Achse radial zur Längserstreckung des Tragstückes 11 und bei der dargestellten Ausführungsform parallel zur Achse L liegt sowie über die der Klemmbefestigung 2 abgewandte Seite des Tragstückes 11 wegsteht. Wenigstens ein Auflageelement 12 ist beispielsweise in Längsrichtung des Tragstückes 11 verstellbar. Weiterhin ist beispielsweise auch das Tragstück 11 um die Längsachse L drehbar bzw. verstellbar an der Klemmbefestigung 2 gehalten.

Zur Verwendung der von der Klemmbefestigung 2, dem Tragstück 11 mit den Auflageelementen 12 gebildeten Auflage 1 wird die Klemmbefestigung 2 an einer in der Figur 1 mit unterbrochener Linie angedeuteten vertikalen Säule oder Stange 13 eines Infustionsständers befestigt. Hierfür wird das Klemmstück 3 mit der Ausnehmung 4 seitlich auf die Stange 13 aufgeschoben, und zwar in der Achsrichtung B radial zur Längserstreckung der Stange 13. Die Klemm-Mutter 9 ist hierbei soweit zurückgedreht, daß die Ausnehmung 4 vollständig frei ist. Nach dem Aufschieben auf die Stange 3 erfolgt mit Hilfe der Klemm-Mutter 9 ein Festklemmen der Klemmbefestigung 2 an dieser Stange 13. Hierfür wird die Mutter 9 soweit vorgedreh, bis sie mit ihrer die Gewindeöffnung 10 umgebenden Stirnfläche 9' gegen die Stange 13 angepreßt anliegt und diese in der Ausnehmung 4 und dabei insbesondere gegen die Seitenfläche 6 bzw. deren Abschnitte 6' und 6'' anliegend festklemmt. Der Querschnitt der Ausnehmung 4 ist in jeder Achse der der senkrecht zur Achse A angeordneten Querschnittsebene größer als der Außendurchmesser der Stange 13.

Die Klemm-Mutter 9 ist an ihrer Umfangfläche bei der darstellten Ausführungsform kreiszylinderförmig ausgebildet und mit einer Rändelung versehen.

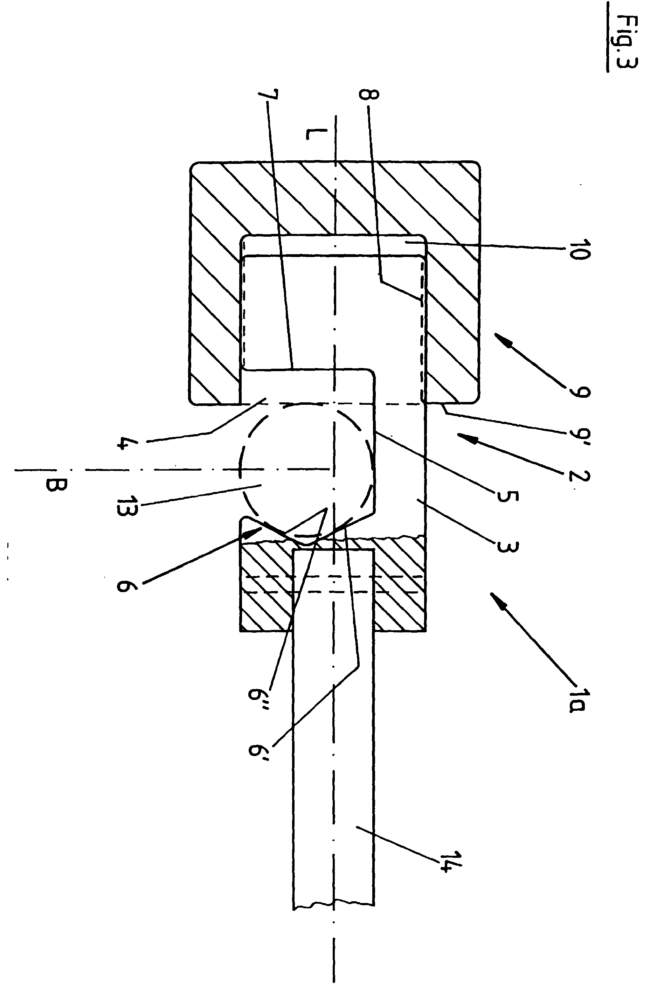

Die vorstehend beschriebene Klemmbefestigung 2 kann auch für die Befestigung anderer Funktionselemente, z.B. an einem Infusionsständer oder einem Bett (Krankenhausbett) usw. dienen. Die Figur 3 zeigt als weitere mögliche Ausführungsform eine Tischablage 1a, die wiederum die Klemmbefestigung 2 bestehend aus dem Klemmstück 3 und der Klemm-Mutter 9 aufweist, wobei an dem der Klemm-Mutter 9 entferntliegenden Ende des Klemmstücks 3 eine Tischplatte 14 befestigt ist, die mit ihren Oberflächenseiten in Ebenen parallel zur Längsachse L und zur Achse A der Ausnehmung 4 liegt. Die Tischplatte 14 ist bei der dargestellten Ausführungsform am Klemmstück 3 dadurch befestigt, daß dieses an dem der Seitenfläche 6 benachbarten Ende eine nutenförmige Ausnehmung aufweist, in die die Tischplatte 4 hineinreicht und in der diese Platte in geeigneter Weise befestigt ist. Die Platte 14 besteht beispielsweise aus Metall oder Kunststoff. Mit Hilfe der Klemmbefestigung 2 kann das Funktionselement bzw. die Ablage 1a an einer Stange eines Netz befestigt werden.

Bei Anbringung der Tischplatte 14 in einer gegenüber der Figur 3 um 90° gedrehten Lage in Bezug auf das Klemmstück 3, d.h. in einer Lage, in der die Ebene der Tischplatte 14 parallel zur Längsachse L, aber senkrecht zur Achse A der Ausnehmung 4 liegt, kann die Ablage 1a auch an vertikalen Stangen, beispielsweise an der Stange 13 des Infusionsständers befestigt werden.

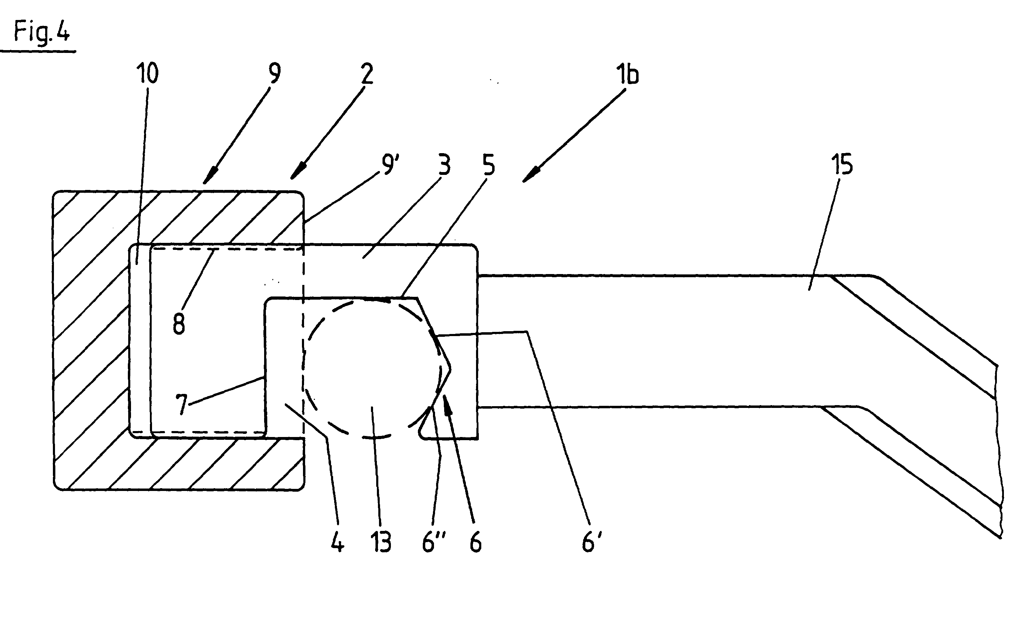

Die Figur 4 zeigt als weitere mögliche Ausführungsform einen Handgriff 1b mit der Klemmbefestigung 2 und mit einem an dieser befestigten Griffelement 15. Dieses ist wiederum an der der Klemm-Mutter 9 entferntliegenden Stirnseite des Klemmstückes 3 in geeigneter Weise befestigt, beispielsweise durch Einschrauben in eine Gewindebohrung des Klemmstücks 3.

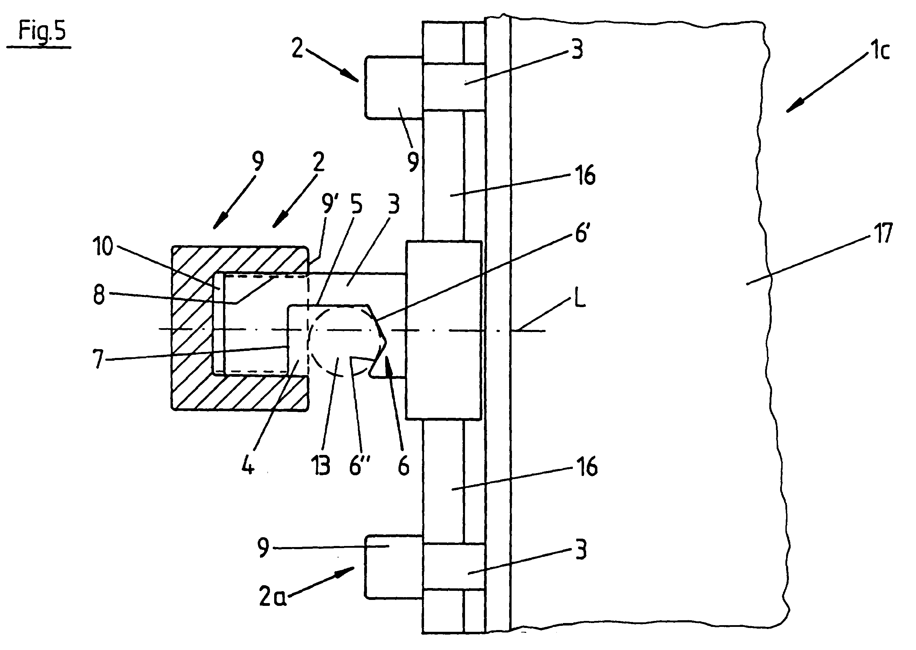

Bei den bisher beschriebenen Funktionselementen 1, 1a und 1b wurde jeweils nur eine einzige Klemmbefestigung 2 verwendet. Die Figur 5 zeigt in Draufsicht eine tischförmige Ablage 1c, die insgesamt drei Klemmbefestigungen 2 bzw. 2a aufweist. Hierbei entspricht die Klemmbefestigung 2 wiederum der Klemmbefestigung 2, wie sie vorstehend im Zusammenhang mit den Figuren 1-4 beschrieben wurde und mit der bei der dargestellten Ausführungsform eine Befestigung der Ablage 1c an einer vertikalen Stange 13 z.B. eines Infusionsständers möglich ist.

An dem der Klemm-Mutter 9 entferntliegenden Stirnseite des Klemmstücks 3 ist eine Stange 16 befestigt, die mit ihrer Achse radial zur Längsachse L der Klemmbefestigung 2 angeordnet ist und beidendig über das Klemmstück 3 wegsteht. An der Stange 16 sind zwei kleinere Klemmbefestigungen 2a durch Festklemmen gehalten, und zwar in der gleichen Weise, wie dies vorstehend für die Klemmbefestigung 2 an der Stange 13 beschrieben wurde, d.h. die Stange 16 greift in die Ausnehmung 4 der jeweiligen Klemmbefestigung 2a ein und ist durch die festgezogene Klemm-Mutter 9 in dieser Ausnehmung fixiert. Die Längsachsen der Klemmstücke 3 der Klemmbefestigungen 2a sind radial zur Längserstreckung der Stange 16 orientiert, und zwar bei der dargestellten Ausführungsform parallel zur Längsachse des Klemmstücks 3 der Klemmbefestigung 2. Weiterhin liegt bei der dargestellten Ausführungsform die Achse der Stange 16 senkrecht oder quer zur Achse A der Ausnehmung 4 der Klemmbefestigung 2.

An den kleineren Klemmbefestigungen 2a bzw. an deren Klemmstücken 3 ist eine Platte 17 befestigt, und zwar beispielsweise in der gleichen Weise, wie dies bei der Ablage 1a für die Tischplatte 14 beschrieben wurde.

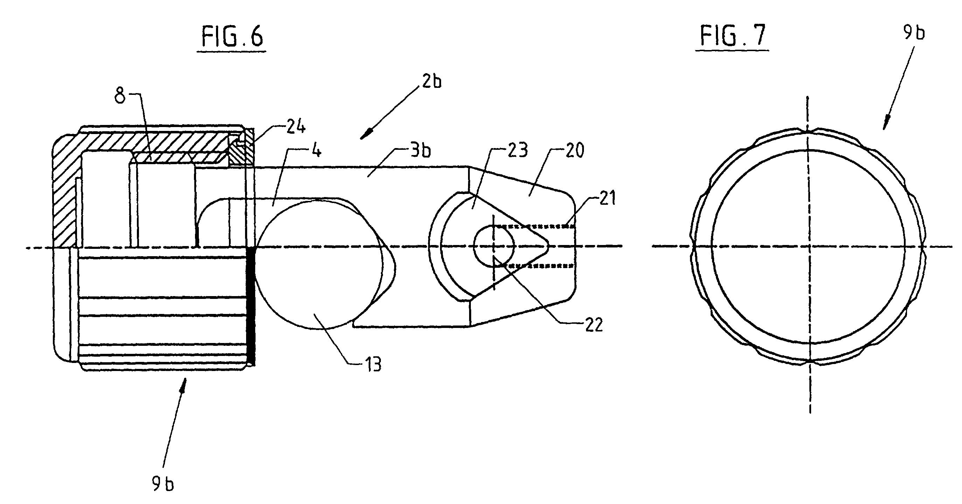

Die Figuren 6 und 7 zeigen als weitere mögliche Ausführungsform eine Klemmbefestigung 2b, die der Klemmbefestigung 2 im wesentlichen entspricht und ebenfalls zur Befestigung von nicht dargestellten Funktionselementen an einer Stange oder Säule 13 dient. Die Klemmbefestigung 2b besteht aus dem dem Klemmstück 3 entsprechenden Klemmstück 3b, welches zusammen mit der Klemm-Mutter 9b das Klemmstück 2b bildet, sich von dem Klemmstück 3 aber dadurch unterscheidet, daß das Klemmstück 3b an seinem der Klemm-Mutter 9b abgewandten Ende bei 20 kegelstumpfförmig sich verjüngend ausgebildet ist und dort mit einer axial verlaufenden Bohrung 21, beispielsweise einer Gewindebohrung sowie einer die Bohrung 21 schneidenden radialen Bohrung 22 ausgeführt ist, so daß an der Klemmbefestigung 2b unter Verwendung der Bohrung 21 beispielsweise ein Handgriff und unter Verwendung der Bohrung 22 beispielsweise eine plattenförmige Ablage befestigt werden kann. Im Bereich der Bohrung 22 ist das Klemmstück 3b zumindest einseitig mit einer planen Fläche 23 versehen, deren Ebene parallel zur Längsachse des Klemmstückes 3b sowie senkrecht zur Achse der Bohrung 22 liegt. Die Anordnung ist weiterhin so getroffen, daß die Achse der Bohrung 22 im Verwendungsfall parallel zur Achse der von der Ausnehmung 4 aufgenommenen Stange oder Säule 13 liegt.

An der dem Ende 20 abgewandten Seite ist das Klemmstück 3b wiederum mit dem Außengewinde 8 für die Klemm-Mutter 9b versehen, Im Bereich dieses Außengewindes 8 besitzt das Klemmstück 3b einen etwas vergrößerten Durchmesser. An der offenen Seite der Klemm-Mutter 9b ist ein Fixier- bzw. Zentrierring 24 vorgesehen, der aus einem verschleißfesten bzw. zähen Kunststoffmaterial besteht und mittels eines angeformten ringförmigen Abschnittes in einer Hinterschneidung oder Nut an der Innenfläche der Klemm-Mutter 9b gehalten ist, und zwar derart, daß derjenige Teil des Ringes 24, der gegen die die offene Seite der Klemm-Mutter 9b umschließende ringförmige Stirnfläche dieser Klemm-Mutter anliegt, die Anlagefläche bildet, mit der die Klemm-Mutter 9b im festgezogenen Zustand gegen die Stange oder Tragsäule 13 anliegt.

Der Ring 24 ist weiterhin so ausgebildet und gehalten, daß er um die Längsachse L der Klemmbefestigung 2b bzw. des Klemmstücks 3b oder der Klemm-Mutter 9b frei drehbar ist. Beim Festziehen der Klemmbefestigung 2b an der Stange oder Säule 13 durch Festdrehen der Klemm-Mutter 9b kommt somit der Ring 24 schließlich zur Anlage gegen die Stange oder Säule 13, wobei dann beim weiteren Festdrehen der Klemm-Mutter 9b der Ring 24 nicht mitdreht, aber geringfügig elastisch verformt wird, so daß ein hochbelastbarer und insbesondere auch sicherer Halt der Klemmbefestigung 2b an der Stange oder Säule 13 erreicht ist.

Da der Innendurchmesser des Ringes 24 kleiner ist als der Außendurchmesser, den das Klemmstück 3b im Bereich des Außengewindes 8 aufweist, ist die Klemm-Mutter 9b durch diesen Ring 24 zugleich auch auf dem Klemmstück 3b gesichert.

Die Erfindung wurde voranstehend an Ausführungsbeispielen beschrieben. Es versteht sich, daß zahlreiche Änderungen sowie Abwandlungen möglich sind, ohne daß dadurch der der Erfindung zugrundeliegende Erfindungsgedanke verlassen wird.

### Bezugszeichenliste

- 1, 1a, 1c: Ablage
- 1b: Handgriff
- 2, 2a: Klemmbefestigung
- 3, 3b: Klemmstück
- 4: Ausnehmung
- 5: Bodenfläche
- 6, 7: Seitenfläche
- 6', 6'': Abschnitt
- 8: Außengewinde
- 9, 9b: Klemm-Mutter
- 9': Stirnfläche
- 10: Muttergewindeöffnung
- 11: Tragstück
- 12: Auflageelement
- 13: Stange oder Säule
- 14: Tischplatte
- 15: Handgriff
- 16: Stange
- 17: Tischplatte
- A, B: Achsrichtung
- L: Längsachse

## Patentansprüche

1. Klemmbefestigung zur Befestigung eines Funktionselementes, beispielsweise einer Auflage, einer Ablage, eines Tisches, eines Handgriffs, **dadurch gekennzeichnet**, daß die Klemmbefestigung (2, 2a) zur Befestigung an einer Stange oder einem stangenartigen Element (13, 16) aus einem Klemmstück (3) besteht, welches eine Ausnehmung (4) aufweist, mit der das Klemmstück (3) in einer ersten Achsrichtung radial zur Längserstreckung des stangenartigen Elementes (13, 16) auf dieses seitlich aufsetzbar ist, so daß das stangenartige Element (13, 16) von der Ausnehmung (4) aufgenommen ist und mit seiner Längserstreckung in einer zweiten Achsrichtung (A) innerhalb der Ausnehmung (4) angeordnet ist, und daß am Klemmstück (3) eine in einem Gewinde (8) des Klemmstücks (3) geführte Klemm-Mutter (9) vorgesehen ist, die in einer dritten Achsrichtung (L) senkrecht oder quer zur zweiten Achsrichtung (A) durch Verdrehen zwischen einem nicht klemmenden Zustand und einem klemmenden Zustand bewegbar ist, in welchem die Klemm-Mutter (9) das in die Ausnehmung (4) eingeführte stangenartige Element in dieser Ausnehmung festklemmt.

2. Klemmbefestigung nach Anspruch 1, dadurch gekennzeichnet, daß die Klemm-Mutter (9) im nicht klemmenden Zustand sich außerhalb der Ausnehmung (4) befindet, und/oder daß die Klemm-Mutter (9) im klemmenden Zustand die Ausnehmung (4) zumindest teilweise abdeckt, und/oder daß die Klemm-Mutter (9) hut- oder napfartig ausgebildet ist.

3. Klemmbefestigung nach einem der vorhergehenden Ansprüche, dadurch gekennzeichnet, daß die Ausnehmung (4) zumindest an einer der Klemm-Mutter (9) gegenüberliegenden Seitenfläche (6) einen konkaven Verlauf aufweist.

4. Klemmbefestigung nach einem der vorhergehenden Ansprüche, dadurch gekennzeichnet, daß das Klemmstück (3) zumindest in einem das Gewinde (8) für die Klemm-Mutter (9) aufweisenden Teil als Zylinderstück mit einer kreiszylinderförmigen Außenfläche ausgebildet ist.

5. Klemmbefestigung nach einem der vorhergehenden Ansprüche, dadurch gekennzeichnet, daß die Ausnehmung (4) am Umfang des Klemmstücks (3) über einen Winkelbereich von etwa 180° offen ist.

6. Klemmbefestigung nach einem der vorhergehenden Ansprüche, dadurch gekennzeichnet, daß sie Teil eines Funktionselementes ist, welches an einem stangenartigen Element, z.B. eines Bettes, beispielsweise eines Krankenhaus- oder Pflegebettes und/oder eines Infusionsständers befestigbar ist, beispielsweise Teil einer Auflage oder Ablage für die Abstützung von Extremitäten des menschlichen Körpers oder eines Handgriffs (1b) oder eines Tisches oder einer Ablage (1a, 1c).

7. Klemmbefestigung nach einem der vorhergehenden Ansprüche, dadurch gekennzeichnet, daß die Klemm-Mutter (9b) eine von einem vorzugsweise frei drehbaren Ring (24) gebildete Anlagefläche aufweist, wobei daß der Ring (24) beispielsweise aus Metall oder Kunststoff besteht.

8. Funktionselement, beispielsweise Auflage oder Ablage für Extremitäten des menschlichen Körpers unter Verwendung einer Klemmbefestigung nach einem der Ansprüche 1 - 7, dadurch gekennzeichnet, daß am Klemmstück (3) ein Tragstück (11) befestigt ist, an welchem wenigstens ein Auflageelement (12) vorgesehen ist.

9. Funktionselement, beispielsweise Tisch oder Tischablage unter Verwendung wenigstens einer Klemmbefestigung (2, 2a) nach einem der Ansprüche 1 - 7, dadurch gekennzeichnet, daß an dem Klemmstück (3) wenigstens eine Ablage oder Tischplatte (14, 17) gehalten ist,
wobei beispielsweise die Ablage oder Tischplatte (14) unmittelbar am Klemmstück (3) gehalten ist oder
beispielsweise am Klemmstück (3) einer ersten Klemmbefestigung (2) ein Tragstück oder eine Stange (16) befestigt ist, an der über wenigstens eine zweite Klemmbefestigung (2a) die Ablage oder Tischplatte (17) befestigt ist.

10. Funktionselement, beispielsweise Handgriff unter Verwendung einer Klemmbefestigung (2) nach einem der Ansprüche 1 - 7, dadurch gekennzeichnet, daß am Klemmstück (3) ein Griffelement (15) befestigt ist.

11. Funktionselement, beispielsweise Infusionsständer mit einer vertikalen Säule (13), gekennzeichnet durch ein an dieser Säule (13) durch eine Klemmbefestigung nach einem der vorhergehenden Ansprüche befestigtes Funktionselement (1, 1a, 1b, 1c).
